# EUROPÄISCHE PATENTSCHRIFT

(11) **EP 1 624 057 B1**
(45) Veröffentlichungstag und Bekanntmachung des Hinweises auf die Patenterteilung: **11.06.2008**
(21) Anmeldenummer: 05090293.1
(22) Anmeldetag: 15.06.1998
(51) Int. Cl.: C12N 9/26, C07K 14/435, A61K 38/47, A61K 35/48, A61P 35/00, A61P 37/02

(54) **Hyaluronidase enthaltendes Präparat und pharmazeutische Verwendung davon**
Pharmaceutical composition containing hyaluronidase and uses thereof
Composition pharmaceutique à base de hyaluronidase et sa utilisation

(30) Priorität: 17.06.1997 DE 19726626
(43) Veröffentlichungstag der Anmeldung: 08.02.2006
(62) Teilanmeldung aus: 98250209.8
(73) Patentinhaber: Riemser Arzneimittel AG, 17493 Greifswald (DE)
(72) Erfinder: von Below, Gudrun, 17493 Greifswald (DE); Schweigle, Bernhard, 06862 Meinsdorf (DE); Göricke, Bernd, 06773 Jüdenberg (DE)
(74) Vertreter: Jungblut, Bernhard Jakob

(56) Entgegenhaltungen:
- WO-A1-96/31596
- WO-A1-98/00553
- US-A- 3 708 575
- DATABASE WPI Section Ch, Week 199534 Derwent Publications Ltd., London, GB; Class B04, AN 1995-262302 XP002155221 & RU 2 027 759 C1 (PAK V N) 27. Januar 1995 (1995-01-27)
- DATABASE WPI Section Ch, Week 199647 Derwent Publications Ltd., London, GB; Class B04, AN 1996-475152 XP002155222 & RU 2 054 943 C1 (KHARK BIOLEK ENTERP) 27. Februar 1996 (1996-02-27)
- YANG C-H ET AL: "PURIFICATION OF BULL SPERM HYALURONIDASE EC-3.2.1.25 BY CONCANAVALIN A AFFINITY CHROMATOGRAPHY" BIOCHIMICA ET BIOPHYSICA ACTA, Bd. 391, Nr. 2, 1975, Seiten 382-387, XP000972379 ISSN: 0006-3002
- MEYER M.F. ET AL: "THE SOLUBLE HYALURONIDASE FROM BULL TESTES IS A FRAGMENT OF THE MEMBRANE-BOUND PH-20 ENZYME" FEBS LETTERS, Bd. 413, 1997, Seiten 385-388, XP000961262 AMSTERDAM, NL ISSN: 0014-5793
- HOBARTH K. ET AL: "TOPICAL CHEMOPROPHYLAXIS OF SUPERFICIAL BLADDER CANCER WITH MITOMYCIN C AND ADJUVANT HYALURONIDASE" EUROPEAN UROLOGY, 21. März 1992 (1992-03-21), Seiten 206-210, XP000570526 BASEL ISSN: 0302-2838
- DATABASE EMBASE [Online] ELSEVIER SCIENCE PUBLISHERS, AMSTERDAM, NL; 1989, MAIER U ET AL: "Metaphylactic effect of mitomycin C with and without hyaluronidase after transurethral resection of bladder cancer: Randomized trial" XP002415853 Database accession no. EMB-1989089181 & JOURNAL OF UROLOGY 1989 UNITED STATES, Bd. 141, Nr. 3 I, 1989, Seiten 529-530, ISSN: 0022-5347
- YUK FUNG HUI ET AL: "Gemcitabine: A cytidine analogue active against solid tumors" AMERICAN JOURNAL OF HEALTH-SYSTEM PHARMACY,XX,XX, Bd. 54, Nr. 2, 15. Januar 1997 (1997-01-15), Seiten 162-170, XP002110340 ISSN: 1079-2082
- VAN MOORSEL C.J.A. ET AL: "Gemcitabine: Future prospects of single agent and combination studies" ONCOLOGIST, Bd. 2, Nr. 3, 1997, Seiten 127-134, XP002110339 ISSN: 1083-7159
- CARMICHAEL J. ET AL: "Advanced breast cancer: Investigational role of gemcitabine" EUROPEAN JOURNAL OF CANCER, Bd. 33, Nr. SUPPL. 01, 1997, Seiten S27-S30, XP005058656 ISSN: 0959-8049

## Beschreibung

Die Erfindung betrifft eine Hyaluronidase enthaltenden Präparats erhältlich durch ein Verfahren, wobei Säugetierhoden, insbesondere Stierhoden, zu einem Brei zekleinert werden, wobei aus dem Brei Proteine und Enzyme im Sauren ein Eluat bildend gelöst werden, wobei das Eluat einer fraktionierten Ammoniumsulfatfällung unterworfen und so eine Hyaluronidasefällungsfraktion erhalten wird, wobei die Hyaluronidasefällungsfraktion einer Dialyse zur Abtrennung von Ammoniumsulfat unterworfen wird, wobei die dialysierte Hyaluronidasefällungsfraktion zur Aufreinigung einer Adsorptions- und Desorptionsverfahrenstufe an Aluminiumoxid-hydrat unterworfen wird und wobei die aufgereinigte Hyaluronidasefällungsfraktion galenisch hergerichtet wird, sowie Verwendungen des so hergestellten Hyaluronidase enthaltenden Präparates. - Als Hyaluronidase wird eine Gruppe von Enzymen bezeichnet, die Hyaluronsäure zu spalten vermag. Hyaluronsäure ist linear aus alternierenden Glucuronsäure- und N-Acetylglucosamin-Resten aufgebaut und weist ein Molekulargewicht von unter 20.000 bis weit über 1.000.000 auf. In einem Organismus bildet Hyaluronsäure insbesondere einen Hauptbestandteil von bestimmten hochmolekularen Proteoglykanen, welche u.a. in Haut-, Knorpel- und Bindegeweben vorkommen. In solchen Proteoglykanen, beispielsweise Chondroitin-4/6-sulfat und Mukotinsulfat bzw. Dermatansulfat, bildet Hyaluronsäure zwar eine zentrale, jedoch nicht kovalent mit dem Protein verbundene Grundeinheit. Hyaluronidasen vermögen durch Spaltung der Proteoglykane Gewebe durchlässig für Fremdsubstanzen zu machen. Sie kommen insbesondere im Akrosom von Spermien vor und ihre Proteoglykane spaltende Wirkung ermöglicht Spermienzellen letzendlich natürlicherweise die Fusion mit einer Eizelle. Daher eignen sich grundsätzlich in Hoden von allen Tierarten enthaltene Spermien zur Gewinnung von Hyaluronidasen in wirtschaftlich brauchbaren Mengen. Insbesondere sind Großschlachtviehhoden brauchbar. Bei einer Fällung von Proteinen werden gelöste Proteine und/oder Enzyme durch Reaktion mit dem Fällungsmittel präzipitiert. Bei einer fraktionierten Fällung macht man sich zunutze, daß verschiedene Proteine und/oder Enzyme eine verschiedene Affinität zu dem Fällungsmittel aufweisen. Dabei werden meist in einer ersten Fällungstufe nicht gewünschte Proteine und/oder Enzyme präzipitiert und abgetrennt, in einer zweiten Fällungsstufe dann aus der verbleibenden Lösung gewünschte Proteine und/oder Enzyme präzipitiert und die so erhaltene Fällungsfraktion der weiteren Verarbeitung zugeführt. Wesentlich bei der fraktionierten Fällung ist die Abstimmung der in den jeweiligen Fällungsstufen eingesetzten Fällungsmittelmengen bezogen auf das jeweilige Ausgangsmaterial bzw. die Menge an eingesetztem Ausgangsmaterial. Diese Abstimmung wird in der Regel über die Fällungsmittelkonzentration in der Proteine und/oder Enzyme enthaltenden Lösung getroffen. Wird beispielsweise in der ersten Fällungsstufe mit einer zu hohen Fällungsmittelkonzentration gearbeitet, so werden störenderweise auch gewünschte Proteine und/oder Enzyme abgetrennt und verloren. Wird dagegen in der ersten Fällungsstufe mit einer zu geringen Fällungsmittelkonzentration gearbeitet, verbleiben störend viele unerwünschte Proteine und/oder Enzyme in Lösung. Analoges gilt für die zweite Fällungsstufe, in der die gewünschten Proteine und/oder Enzyme präzipitiert werden sollen. Der Ausdruck Dialyse bezeichnet die Trennung gelöster Moleküle nach ihrer Größe infolge von Diffusion bzw. Retention durch eine semipermeable Membran. Die Dialyse wird insbesondere zur Abtrennung hochmolekularer Stoffe, wie Proteine und Enzyme, von niedermolekularen Stoffen, wie Salze, Monosaccharide, Aminosäure etc., eingesetzt. Bei einer Aufreinigung durch Adsorption und Desorption handelt es sich um eine Anreicherungsmethode, die sich verschiedene Adsorptionseigenschaften von verschiedenen Adsorptiven an einem Adsorbens zu Nutze macht. Durch Wahl des Adsorbens sowie Einstellung bestimmter Adsorptionsbedingungen werden bestimmte Zielsubstanzen bevorzugt adsorbiert und/oder desorbiert und so angereichert, während unerwünschte Substanzen entweder nicht adsorbiert und mit der Flüssigphase abgetrennt und/oder nicht desorbiert und mit der Festphase abgetrennt werden. Der Ausdruck der galenischen Herrichtung bezeichnet die Verarbeitung einer Substanz zu einem Präparat, welches unmittelbar oder nach Lösung, beispielsweise in physiologischer Kochsalzlösung, einem Organismus zugeführt werden kann, ohne daß darreichungsbedingte Verträglichkeitsprobleme entstehen.

Aus der Praxis ist es bekannt, Hyaluronidase durch Schwermetallfällung aufzureinigen. Diese Vorgehensweise erfordert allerdings die besonders sorgfältige Entfernung aller Schwermetallionen aus dem erhaltenen Präparat aufgrund derer Toxizität. Weiterhin ist es aus der Praxis bekannt, Hyaluronidase durch fraktionierte Ethanolfällung zu erhalten. Hierbei sind nicht ohne weiteres pyrogenfreie Präparate erhältlich. Schließlich ist die Aufreinigung mittels verschiedener Adsorbentien, beispielsweise Aktivkohle, Calciumphosphat oder Aluminiumhydroxyd, bekannt. Dabei befriedigt aber die erreichbare Ausbeute nicht.

Ein Verfahren der eingangs genannten Art ist aus der Literaturstelle DD-PS-24229 bekannt. Im Rahmen des insofern bekannten Verfahrens wird im Anschluß an eine Ammoniumsulfatfällung mit zwei Adsorptions- und Desorptionsverfahrenstufen mit Aluminiumoxid-hydrat unter Verwendung differenter Puffersysteme gearbeitet. Im Rahmen der Ammoniumsulfatfällung wird in der ersten Fällungsstufe mit einer 30%igen Ammoniumsulfatkonzentration und in der zweiten Fällungsstufe mit einer 70%igen Ammoniumsulfatkonzentrationgearbeitet. Hierbei wird ein pyrogenfreies Präparat mit unbefriedigender Ausbeute erhalten, zudem ist der Aufwand störend hoch.

Demgegenüber liegt der Erfindung das technische Problem zugrunde, eine Hyaluronidase enthaltenden Präparates anzugeben, welches mit guter Wirtschaftlichkeit hinsichtlich Aufwand und Ausbeute auszuführen ist, und welches ein Präparat liefert, das bei Anwendung in der Medizin besonders wirksam ist.

Zur Lösung dieses Problems lehrt die Erfindung, daß die fraktionierte Ammoniumsulfatfällung in einer ersten Fällungsstufe in einer 10-29%igen, vorzugsweise einer 12-25 Gew.-%igen, wäßrigen Ammoniumsulfatlösung und in einer zweiten Fällungsstufe in einer 15-65 Gew.-%igen, vorzugsweise einer 25-50 Gew.-%igen, wäßrigen Ammoniumsulfatlösung durchgeführt wird und daß die aufgereinigte Hyaluronidasefällungsfraktion vor der galenischen Herrichtung einer Ultrafiltration mit einer Ausschlußgrenze von 5.000 bis 30.000 Dalton, vorzugsweise bis 15.000 Dalton, unterzogen wird. - Der Ausdruck Ultrafiltration bezeichnet ein Filterverfahren, bei welchem unter Druck größere Partikel und/oder Moleküle von Lösungsmitteln und kleineren Molekülen getrennt werden. Ein Ultrafilter ist dabei durch die Ausschlußgrenze gekennzeichnet, welche angibt, bis zu welcher Molekularmasse Moleküle durch die Filtermembran gelassen werden. Der Ausdruck Gew.-% meint g Ammoniumsulfat je 100g Ammoniumsulfat-Lösung.

Überraschenderweise wird mit dem erfindungsgemäßen Verfahren ein Präparat erhalten, das sich durch eine besonders hohe physiologische Wirksamkeit auszeichnet, und dennoch vergleichsweise einfach und kostengünstig herstellbar ist. Von besonderer Bedeutung ist dabei die Erkenntnis, daß die physiologische Wirksamkeit nicht eine monotone Funktion der Reinheit des Präparates ist. Vielmehr wurde gefunden, daß ein Hyaluronidase enthaltendes Präparat extrem hoher Reinheit eine gegenüber einem erfindungsgemäß hergestellten Präparat verringerte physiologische Wirkung hat. Dies kann mehrere Gründe haben. Zum einen kann bei extrem hoher Reinheit die stabilisierende Wirkung fremder (inaktiver) Proteine fehlen, was wiederum zu einer geringeren Aktivität der Hyaluronidase selbst führt. Eine solche Komplexierung kann auch zu einer deutlich höheren Verweilzeit der Hyaluronidase im Organismus führen. Zum anderen kann nicht ausgeschlossen werden, daß bei einer extremen Reinheit der Hyaluronidase fremde (inaktive) Proteine abgetrennt worden sind, die als Adjuvans für die Hyaluronidase zu wirken vermögen. Ein erfindungsgemäß hergestelltes Präparat zeigt jedoch hinsichtlich verschiedener physiologischer Wirkungen optimale Aktivität, was vermutlich insbesondere auf die im Vergleich zur Literaturstelle DD-PS-24229 relativ enge Konzentrationabstufung im Rahmen der fraktionierten Ammoniumsulfatfällung zurückzuführen ist.

Im einzelnen bestehen verschiedene Möglichkeiten der Ausführung des erfindungsgemäßen Verfahrens. Folgende Möglichkeiten sind bevorzugt.

Die Lösung im Sauren der Proteine und Enzyme aus dem Brei kann mittels 0,02-0,2 molarer, vorzugsweise 0,1 molarer Essigsäure durchgeführt werden, wobei das Gewichtsverhältnis Essigsäure zu Stierhoden 0,2:1 bis 5:1, vorzugsweise 0,8:1 bis 1,2:1, höchstvorzugsweise ca. 1:1, beträgt, wobei die Mischung aus Brei und Essigsäure zumindest 2 Stunden, vorzugsweise mehr als 4 Stunden, gerührt wird und wobei das danach erhaltene Eluat durch Zentrifugation oder Filtration von ungelöstem Festkörper abgetrennt wird.

Vorteilhafterweise wird die fraktionierte Ammoniumsulfatfällung in der ersten Fällungsstufe in einer 15-20 Gew.-%igen, vorzugsweise einer ca. 17,5 Gew.-%igen, wäßrigen Ammoniumsulfatlösung und in einer zweiten Fällungsstufe in einer 30-40 Gew.-%igen, vorzugsweise einer ca. 34,3 Gew.-%igen, wäßrigen Ammoniumsulfatlösung durchgeführt. In der ersten Fällungstufe ausgefällte, unerwünschte Proteine und Enzyme und die in der zweiten Fällungsstufe erhaltene Hyaluronidasefällungsfraktion können mittels Zentrifugation oder Filtration von der Flüssigphase abgetrennt werden.

Die Hyaluronidasefällungsfraktion wird im einzelnen vorzugsweise in Wasser suspendiert und die Dialyse wird bis zu einer Osmolalität von 10-40 mosm/kg, vorzugsweise von 20-30mosm/kg, durchgeführt. Der Proteingehalt der dialysierten Hyaluronidasefällungsfraktion wird auf 1,0-4,0%, vorzugsweise 1,9-2,1%, gemessen nach Kjeldahl, eingestellt. Der pH wird danach auf 4,5-5,8, vorzugsweise ca. 5,1, eingestellt und entstehendes Präzipitat abzentrifugiert. Die Osmolalität ist definiert durch die Gleichung m = (-ln a_{A})/M_{A}, wobei m die Osmolalität in Mol/kg (bzw. mosm/kg), a_{A} die Aktivität des Lösungsmittels A (hier Wasser) und M_{A} die Molmasse des Lösungsmittels A sind. Die Bestimmung des Proteingehaltes nach Kjeldahl ist im Kern die bekannte Kjeldahl-Stickstoffbestimmung, aus welcher auf den Proteingehalt zurückgerechnet werden kann. Hierzu wird beispielhaft auf die Literaturstelle Römpp, Chemie Lexikon, 1995, Paperback Ausgabe, Band 3, S. 2246, sowie die darin genannten Zitate verwiesen.

Im weiteren ist es bevorzugt, wenn der Proteingehalt der in der Adsorptionsverfahrenstufe eingesetzten Hyaluronidasefällungsfraktion auf 1,0-2,0%, vorzugsweise 1,4-1,6%, gemessen nach Kjeldahl, eingestellt wird, dann 1/5-1/20, vorzugsweise 1/10, des Volumens eingesetzter Hyaluronidasefällungsfraktion an Zitratpuffer (pH 8,0) zugegeben werden, danach die Adsorptionsverfahrensstufe durch Zugabe einer 1,0-10%igen, vorzugsweise einer 2,5-3,5%igen, Aluminiumoxid-hydrat Lösung durchgeführt wird, wobei die Menge an zugesetzter Aluminiumoxid-hydrat Lösung 10-50ml/g Protein, vorzugsweise 20-40ml/g Protein, höchstvorzugsweise ca. 30 ml/g Protein, beträgt, und nach der Adsorptionsverfahrenstufe das erhaltene Sediment zumindest einer Waschung, vorzugsweise 2 Waschungen, bei pH 7,5-9,5, vorzugsweise bei ca. pH 8,5, unterworfen wird und dann durch Aufrühren in wäßriger Lösung mit einem pH von 3,5-6,0, vorzugsweise einem pH von ca. 4,2, die Desoptionsverfahrenstufe durchgeführt wird. Die Waschungen werden vorzugsweise mit einem Waschpuffer aus einem Teil Zitratpuffer (pH 8,0) und 1-50, vorzugsweise 10 Teilen (bezogen auf die Gewichte) Wasser durchgeführt, wobei das Volumen des Waschpuffers jeder Waschung dem Gesamtvolumen aus Hyaluronidase + Zitratpuffer entspricht und wobei nach jedem Aufrühren des Sediments im Waschpuffer der pH mit 0,1m Zitronensäure auf ca. 8,5 eingestellt wird.

Weiterhin bevorzugt ist es, wenn die Ultrafiltration mit einer Ausschlußgrenze von 9.000-11.000 Dalton, vorzugsweise von 10.000 Dalton, durchgeführt wird und wenn die Ultrafiltration mit der Maßgabe durchgeführt wird, daß die erhaltene Hyarulonidase-Aktivität mindestens 3000 I.E./ml, vorzugsweise mindestens 5500 I.E./ml, beträgt. Die Bestimmung der Hyaluronidase-Aktivität erfolgt mit einem im Ausführungsbeispiel näher erläuterten Test. Anschließend an die Ultrafiltration kann eine Dialyse bis zu einer Osmolalität von 5-50 mosm/kg, vorzugsweise von 10-30mosm/kg durchgeführt werden.

In Hinblick auf die Abtrennung von pathogenen Keimen und die Vermeidung des Entstehens von Pyrogenen ist es vorteilhaft, wenn anschließend an die Dialyse zur Ammoniumsulfatabtrennung und/oder vor der Ultrafiltration eine Sterilfiltration durchgeführt wird. Bei einer Sterilfiltration wird ein Filter eingesetzt, dessen Porengröße die Retention von Bakterien und ggf. auch Viren gewährleistet.

Von selbstständiger Bedeutung im Rahmen der Erfindung sind die Verwendung eines erfindungsgemäß hergestellten, Hyaluronidase enthaltenden Präparates zur Herstellung eines Arzneimittels zur Behandlung von Erkrankungen, die durch Plaquebildung in Blutgefäßen hervorgerufen sind, sowie die Verwendung eines erfindungsgemäß hergestellten, Hyaluronidase enthaltenden Präparates zur Herstellung eines Arzneimittels oder eines Arzneimittelpakets zur Behandlung von Tumorerkrankungen, wobei das Hyaluronidase enthaltende Präparat mit einem oder mehreren üblichen Zytostatika gemischt und galenisch hergerichtet wird oder wobei das Hyaluronidase enthaltende Präparat sowie eines oder mehrere Zytostatika getrennte und getrennt zu verabreichende Komponenten des Arzneimittelpakets bilden. Als Arzneimittelpaket wird dabei eine sogenannte Kombipackung bezeichnet, in welcher räumlich getrennt zwei verschiedene Wirkstoffpräparate verpackt sind, deren Wirkstoffpräparate jedoch zur gleichzeitigen oder zeitversetzten Verabreichung bei einer medizinischen Indikation bestimmt sind. In letzterem Fall ist kann es bevorzugt sein, wenn das Hyaluronidasepräparat zur Verabreichung vor dem Zytostatikum bestimmt ist. Als Zytostatika werden Substanzen bezeichnet, die Zellen, insbesondere Tumorzellen abzutöten vermögen, zumindest aber proliferationshemmend wirken.

Als Erkrankungen, die durch Plaquebildung in Blutgefäßen hervorgerufen werden, werden beispielhaft genannt: Arteriosklerose, insbesondere der Herzkranzgefäße, der Carotis und der hirnzuführenden Arterien; in schweren Fällen führt dies zu den Krankheitsbildern des Herzinfarkts, der Apoplexie, Durchblutungsstörungen der Extremitäten, des Bindegewebes sowie von sonstigen Organen, wie Hoden etc. Diese Erkrankungen werden in der Regel durch innenwandige Ablagerungen in Blutgefäßen verursacht, aufgrund welcher der Durchfluß durch die Blutgefäße reduziert ist. Diese innenwandigen Ablagerungen werden als Plaque bezeichnet. Plaque läßt sich auf mechanischem Wege, beispielsweise mittels Herzkatether, aber auch auf chemischem oder biochemischem Wege abbauen. In letzterem Fall werden die Substanzen, aus denen Plaque besteht (beispielsweise hyalin-kalkige Ablagerungen), zersetzt und so zumindest teilweise in Lösung gebracht. Überraschenderweise wurde in Durchflußuntersuchungen an extrahierten Blutgefäßen, welche durch Plaque verengt sind, gefunden, daß durch den Einsatz eines erfindungsgemäß hergestellten, eine Hylauronidase enthaltenden Präparats im Durchströmungsmedium eine beachtliche Verbesserung des Durchflußwiderstandes erhalten wird. Dies ist auf die Lösung von Plaque durch Hyaluronidase-Wirkung zurückzuführen. In ersten klinischen Untersuchungen wurde zudem gefunden, daß sich der Zustand von Patienten, welche an plaquebedingten Herz-Kreislauf-Beschwerden leiden, nach Gabe des erfindungsgemäß hergerstellten Präparats beachtlich verbessert. Die Gaben wurde durchgeführt durch i.V.-Injektion von 1500 Einheiten des Präparats, gelöst in physiol. Kochsalzlösung, dreimal pro Woche. Ebenfalls möglich ist die Gabe durch i.p.- oder i.m.-Injektion, sowie die Wahl anderer Konzentrationen bzw. Dosen. Auch der Einsatz zur Prophylaxe ist möglich.

Bei der Verwendung eines erfindungsgemäß hergestellten Präparats zur Herstellung eines Arzneimittels bzw. Arzneimittelpakets zur Behandlung von Tumorerkrankungen wurde überraschenderweise gefunden, daß dieses spezielle Präparat eine besonders gute Wirkung als Adjuvans für verschiedenste Zytostatika aufweist. Dies beruht vermutlich auf der Gewebe durchlässig machenden Wirkung der Hyaluronidase. Als geeignete Antineoplastika bzw. Zytostatika werden beispielhaft genannt: Perfosamid, Cyclophosphamid, Lomustin, Mechlorethamin, Prednison, Vincristin, Methotrexat, Procarbazin, Peptichemio, Doxorubicin, Vindesin, Cisplatin und Mitomycin (C). Besonders bevorzugt kommen jedoch Zytostatika aus der Gruppe der Pyrimidinantagonisten, insbesondere Gemcitabine, zum Einsatz. Die genannten Zytostatika sind frei erhältlich und käuflich erwerbbar. Nach ersten Versuchen ist die Verwendung eines erfindungsgemäßen Präparats in Verbindung mit Gemcitabine zur Behandlung von kolorektalen Karzimonen besonders erfolgversprechend, und zwar insbesondere auch bei Patienten, die 5-FU/Leucovorin-resistent sind. Dabei wird Gemcitabine in Mischung mit einem erfindungsgemäßen Präparat oder separat in einer Dosis von 200 bis 5000 , vorzugsweise 500 bis 4000 mg/m², höchstvorzugsweise 1000 mg/m², an den Tagen 1,8,15, gefolgt von 1 Woche Pause, dann wieder beginnend, über einen Zeitraum von 30-60 min. durch Infusion verabreicht. Die damit bzw. 1/2 bis 3h davor jeweils verabreichte Hyaluronidasemengen betragen 100 bis 4000 I.E., vorzugsweise 300 bis 1000 I.E. Hinsichtlich der Chronologie der Verabreichung sind selbstverständlich Variationen möglich.

Weiterhin ist es möglich, ein erfindungsgemäß hergestelltes Präparat als Arzneimittel zur Tumortherapie bzw. Tumorprophylaxe ohne Beigabe weiterer Substanzen zu verwenden. Insbesondere ist die Beigabe eines Zytostatikums nicht zwingend erforderlich. Dies beruht auf der Erkenntnis, daß das erfindungsgemäße Präparat durch Abbau von Hyaluronsäure bei Tumorzellen diese gleichsam zu demaskieren vermag mit der Folge, daß das körpereigene Immunsystem die insofern demaskierten Tumorzellen zu erkennen und abzutöten vermag.

Im folgenden wird die Erfindung anhand eines Herstellungsbeispiels näher erläutert. Verwendet wurden dabei folgende Puffer: Zitratpuffer pH 8,0 aus 2,80 Vol.-% 0,1m Zitronensäure und 97,20 Vol.-% 0,2m Dinatriumhydrogenphosphat; Natriumacetatpuffer pH 4,2 aus 3,33 Vol.-% 1m Natriumacetat, 6,66 Vol.-% 1m Essigsäure und 90,01 Vol.-% Wasser. Sofern Wasser in einem Puffer oder in anderen Zusammenhängen des Ausführungsbeispiels eingesetzt wird, so handelt es sich um den Reinheitsgrad "zur Injektion".

Als Testsystem für die Bestimmung der Hyaluronidase-Aktivität in Zwischen- und Endprodukten wird der Trübungsverminderungstest benutzt. Die Bestimmung beruht auf der Wirkung der Hyaluronidase, Hyaluronsäure zu hydrolysieren. Hyaluronsäure ergibt mit geeigneten Substanzen wie z.B. Pferdenormalserum oder Albuminlösung eine Trübung. Die Abnahme der Trübung im Reaktionsgemisch wird spektralfotometrisch verfolgt (Trübungsverminderungstest). Die Zwischenprodukte werden bei Bedarf mit Phosphatpuffer vorverdünnt, lyophylisierte Endprodukte mit physiologischer Kochsalzlösung angelöst (Bedingungen: Raumtemperatur). Anschließend werden sie mit der Testlösung (30mg Hyaluronsäure in 25ml bidest. Wasser angelöst und am Tag der Bestimmung im Verhältnis 1+2 mit Phosphatpuffer verdünnt) versetzt. Zusätzlich wird Serumgebrauchslösung zugegeben. Die Menge an Hyaluronsäure wird so gewählt, daß ein Überschuß gegenüber der zu bestimmenden Menge an Enzym eingesetzt wird. Die Trübungsminderung wird anhand der Abnahme der Extinktion bei 640nm photometrisch bestimmt. Die Aktivität wird durch die erhaltene Extinktion anhand der Bezugskurve (ergibt sich aus Standardverdünnungen) errechnet. Als Standard wird ein interner Standard verwendet, welcher mit der Zusammensetzung des Endprodukts übereinstimmt und dessen Aktivität anhand eines WHO-Standards in Mehrfachbestimmungen ermittelt wurde. Die Aktivität des WHO-Standards wird anhand eines geeigneten Londoner Urstandards (Internationaler Standard) bestimmt.

Zunächst werden 30kg enthäutete und verputzte Stierhoden in halbgefrorenem Zustand mit einem Fleischwolf zerkleinert. Der erhaltene Brei wird mit 301 0,1m Essigsäure mindestens 4 Stunden gerührt, wobei Proteine und/oder Enzyme in Lösung gehen. Das Eluat wird durch Zentrifugation oder Filtration abgetrennt. Der Rückstand wird verworfen.

Das so erhaltene Eluat wird dann einer fraktionierten Ammoniumsulfatfällung unterworfen. Hierzu wird es unter Rühren mit 212g Ammoniumsulfat pro Liter versetzt und nach dessen Auflösung noch 30min. nachgerührt. Hieraus ergibt sich eine ca. 17,5 Gew.-%ige Ammoniumsulfatlösung (unter der Annahme, daß die Dichte des Extrakts ca. 1 ist). Der Niederschlag wird abzentrifugiert oder abfiltriert und verworfen. Das erhaltene Filtrat bzw. Zentrifugat wird dann mit 282g Ammoniumsulfat pro Liter versetzt und 30min. nachgerührt. Hieraus ergibt sich eine ca. 34,3 Gew.-%ige Ammoniumsulfatlösung (unter der Annahme, daß die Dichte des Extrakts aufgrund des bereits enthaltenen Ammoniumsulfats ca. 1,1009 ist). Man läßt über Nacht die Fällung absetzen. Die Abtrennung des Niederschlags erfolgt durch Filtration bzw. Zentrifugation, wobei die Hyaluronidasefällungsfraktion als Festkörper erhalten wird. Die Hyaluronidasefällungsfraktion wird in ca. 41 Wasser suspendiert bzw. gelöst und dialysiert. Die Dialyse erfolgt bis zu einer Osmolalität von 20-30mosm/kg. Der Proteingehalt nach Dialyse wird auf 1,9-2,1% (nach Kjeldahl) durch Zugabe von Wasser eingestellt. Der pH der so erhaltenen Lösung wird mit 1n NaOH auf 5,1 gebracht. Nach Zentrifugation des ausdialysierten Ammoniumsulfatpräzipitats erfolgt eine Sterilfiltration.

Das so erhaltene Rohpräparat (Hyaluronidasefällungsfraktion) wird auf einen Proteingehalt von 1,4-1,6% (nach Kjeldahl) verdünnt und anschließend mit 1/10 seines Volumens Zitratpuffer versetzt. Nach Zugabe von Aluminiumoxid-hydrat (30ml/g Protein) einer 2,5 bis 3,5%igen Lösung wird der pH auf 8,5 eingestellt, 30min. gerührt und anschließend zentrifugiert. Der Überstand wird abdekantiert und verworfen. Anschließend erfolgen zwei Waschungen des Präzipitats mit Waschpuffer (1 Teil Zitratpuffer und 10 Teile Wasser). Das Volumen des Waschpuffers jedes Waschgangs entspricht dabei dem Gesamtvolumen aus Rohpräparat und Zitratpuffer. Dabei wird nach jedem Aufrühren des Sediments in Waschpuffer der pH mit 0,1m Zitronensäure auf 8,5 eingestellt. Die Zentrifugations-überstände jeder Waschung werden verworfen. Das Sediment wird dann im doppelten Volumen Natriumacetatpuffer aufgerührt und der pH durch Zugabe von Essigsäure (konz.) auf 4,2 eingestellt. Nach Rühren (30min.) erfolgt die Zentrifugation des Desorptionsgemischs. Der abdekantierte Überstand wird wie folgt weiterverarbeitet und das Sediment verworfen.

Die insofern aufgereinigte Hyaluronidasefällungsfraktion wird dann durch Tiefenfilter (Schichten der Fa. Seitz, Typ BIO 10) sterilfiltriert und danach mit einem Ultrafilter mit einer Ausschlußgrenze von 10.000 Dalton (Hohlfaserpatronen bzw. Kassettensystem der Fa. Millipore) bis zu einer etwaigen Mindestaktivität von 5.500 I.E./ml eingeengt. Dann wird bei konstantem Volumen bis zu einer Osmolalität von 10-30mosm/kg dialysiert. Vor der Weiterverarbeitung wird eine Probe zur Protein- und Aktivitätsbestimmung entnommen und das Verhältnis I.E. Hyaluronidase/mg Protein berechnet (Proteinbest. nach Kjeldahl).

Pro Liter so erhaltenen Produkts werden 0,26 Liter Gelatine-Partialhydrolysat - 3,6%ig zugegeben, so daß die Mischung 7,5 mg Gelatine-Partialhydrolysat pro ml enthält. Hieran schließt sich eine Sterilfiltration an. Dann kann nach Maßgabe der gewünschten Aktivität je Verpackungseinheit die Abpackung erfolgen. Hierbei wird die dafür erforderlich Menge Gelatine-Partialhydrolysat bzw. Wasser zugesetzt, wobei stets eine gewisse Menge Gelatine-Partialhydrolysat als Schutzkolloid zugegen sein sollte. Die Abfüllung erfolgt schließlich beispielsweise über 0,2µm Membranfilter oder Filterkerzen in 1ml Ampullen. Sofort nach Abfüllung werden die Ampullen eingefroren und lyophilisiert. Das Abschmelzen der Ampullen sollte zügig geschehen und mit der Maßgabe, daß die Restfeuchte im eingeschlossenen Präparat unterhalb 5% liegt (Trocknungsverlust nach DAB10).

## Patentansprüche

1. Hyaluronidase enthaltendes Präparat, erhältlich durch ein Verfahren, wobei Säugetierhoden, insbesondere Stierhoden, zu einem Brei zerkleinert werden, wobei aus dem Brei Proteine Enzyme im Sauren ein Eluat bildend gelöst werden, wobei das Eluat einer fraktionierten Ammoniumsulfatfällung unterworfen und so eine Hyaluronidasefällungsfraktion erhalten wird, wobei die Hyaluronidasfällungsfraktion einer Dialyse zur Abtrennung von Ammoniumsulfat unterworfen wird, wobei die dialysierte Hyaluronidasefällungsfraktion zur Aufreinigung einer Adsorptions- und Desorptionsverfahrenstufe an Aluminiumoxid-hydrat unterworfen wird, wobei die aufgereinigte Hyaluronidasefällungsfraktion galenisch hergerichtet wird, wobei die fraktionerte Ammoniumsufatfällung in einer ersten Fällungsstufe in einer 10-29 Gew.%-igen wässrigen Ammoniumsulfatlösung und in einer zweiten Fällungsstufe in einer 15-65 Gew.%-igen wässrigen Ammoniumsulfatlösung durchgeführt wird und wobei die aufgereinigte Hyaluronidasefällungsfraktion vor der galenischen Herrichtung einer Ultrafiltration mit einer Ausschlussgrenze von 5.000 bis 30.000 Dalton unterzogen wird.

2. Verwendung des Präparats Anspruch 1 zur Herstellung eines Arzneimittels zur Therapie oder Prophylaxe von Erkrankungen, die durch Plaquebildung in Blutgefäßen hervorgerufen sind.

3. Verwendung nach Anspruch 2 zur Therapie oder Prophylaxe von Arteriosklerose, beispielsweise der Herzkranzgefäße, der Carotis und der hirnzuführenden Arterien, des Herzinfarkts, der Apoplexie, und von Durchblutungsstörungen der Extremitäten und Durchblutungsstörungen des Bindegewebes.

4. Verwendung nach dem vorhergehenden Anspruch, wobei das Arzneimittel zur Injektion i.v., i.p. oder i.m. hergerichtet ist.

5. Verwendung der Hyaluronidase gemäß Anspruch 1 zur Herstellung eines Arzneimittels zur Tumortherapie oder zur Tumorprophylaxe, insbesondere zur Prophylaxe oder Therapie von kolorektalen Karzinomen.

6. Verwendung nach Anspruch 5, wobei das Arzneimittel als Adjuvans für Zytostatika hergerichtet ist.

7. Arzneimittelpaket umfassend eine Hyaluronidase nach Anspruch 1 sowie ein Zytostatikum oder mehrere Zytostatika.

8. Arzneimittelpaket nach Anspruch 7, wobei das Zytostatikum ausgewählt ist aus der Gruppe bestehend aus Perfosamid, Cyclophosphamid, Lomustin, Mechlorethamin, Predinson, Vincristin, Methotrexat, Procarbazin, Peptidchemio, Doxorubicin, vindesin, Cisplatin und/oder Mitomycin.

9. Arzneimittelpaket nach Anspruch 7 oder 8, wobei das Zytostatika aus der Gruppe der Pyrimidinantagonisten ausgewählt ist, insbesondere Gemcitabin ist.

## Claims

1. A preparation containing a hyaluronidase obtainable by a method, wherein mammal testicles, in particular bull testicles are broken up to a paste, wherein from the paste proteins and enzymes are separated in an acid condition, forming an eluate, wherein the eluate is subjected to a fractioned ammonium sulfate precipitation, and thus a hyaluronidase precipitation fraction is obtained, wherein the hyaluronidase precipitation fraction is subjected to a dialysis for separating ammonium sulfate, wherein the dialyzed hyaluronidase precipitation fraction is subjected for purifying purposes to an adsorption and desorption step at aluminum oxide hydrate, wherein the purified hyaluronidase precipitation fraction is galenically prepared, wherein the fractioned ammonium sulfate precipitation is performed in a first precipitation step in a 10 - 29 %-w/w aqueous ammonium sulfate solution and in a second precipitation step in a 15 - 65 %-w/w aqueous ammonium sulfate solution, and wherein the purified hyaluronidase precipitation fraction is subjected before the galenic preparation to an ultrafiltration with an exclusion limit of 5,000 to 30,000 Daltons.

2. The use of the preparation according to claim 1 for producing a drug for the therapy or prophylaxis of diseases, which are caused by plaque formation in blood vessels.

3. The use according to claim 2 for the therapy or prophylaxis of arteriosclerosis, for instance of the coronary vessels, the carotid and the arteries bringing blood to the brain, the heart infarct, the apoplexy, and disturbances of the blood circulation of the extremities and disturbances of the blood circulation of the connective tissue.

4. The use according to the preceding claim, wherein the drug is prepared for the i.v., i.p. or i.m. injection.

5. The use of the hyaluronidase according to claim 1 for producing a drug for the tumor therapy or tumor prophylaxis, in particular for the prophylaxis or therapy of colorectal carcinomas.

6. The use according to claim 5, wherein the drug is prepared as an adjuvant for cytostatics.

7. A drug package containing a hyaluronidase according to claim 1 and one cytostatic or several cytostatics.

8. The drug package according to claim 7, wherein the cytostatic is selected from the group composed of perfosamide, cyclophosphamide, lomustine, mechlorethamine, prednisone, vincristine, methotrexate, procarbazin, peptichemio, doxorubicin, vindesine, cisplatin and/or mitomycin.

9. The drug package according to claim 7 or 8, wherein the cytostatic is selected from the group of the pyrimidine antagonists, in particular gemcitabine.

## Revendications

1. Préparation comprenant une hyaluronidase, que l'on peut obtenir par un procédé, dans lequel des testicules d'un mammifère, en particulier des testicules d'un taureau sont moulus à une pâte, des protéines et des enzymes sont séparées dans l'état acide de la pâte et forment un éluat, l'éluat est soumis à une précipitation fractionnée de sulfate d'ammonium et donc une fraction de précipitation de hyaluronidase est obtenue, la fraction de précipitation de hyaluronidase est soumise à une dialyse pour la séparation de sulfate d'ammonium, la fraction de précipitation de hyaluronidase dialysée est soumise pour la purification à une étape d'adsorption et désorption à un oxyde hydraté d'aluminium, la fraction de précipitation de hyaluronidase purifiée est préparée galéniquement, la précipitation de sulfate d'ammonium fractionnée est exécutée en une première étape de précipitation dans une solution de sulfate d'ammonium aqueuse de 10 29 % en poids, et en une deuxième étape de précipitation dans une solution de sulfate d'ammonium aqueuse de 15 - 65 % en poids, et la fraction de précipitation de hyaluronidase purifiée est soumise avant la préparation galénique à une ultrafiltration avec une limite d'exclusion de 5.000 à 30.000 Daltons.

2. L'utilisation de la préparation selon la revendication 1 pour la production d'un médicament pour la thérapie ou la prophylaxie de maladies causées par la formation d'une plaque dans les vaisseaux sanguins.

3. L'utilisation selon la revendication 2 pour la thérapie ou la prophylaxie de l'athérosclérose, par exemple des vaisseaux coronaires, de la carotide et des artères du cerveau, de l'infarctus du myocarde, de l'apoplexie, d'une mauvaise irrigation sanguine des extrémités et d'une mauvaise irrigation sanguine du tissu conjonctif.

4. L'utilisation selon la revendication précédente, dans laquelle le médicament se fait par injection IV, IP ou IM.

5. L'utilisation de la hyaluronidase selon la revendication 1 pour la production d'un médicament pour la thérapie des tumeurs ou pour la prophylaxie des tumeurs, en particulier pour la prophylaxie ou thérapie de carcinomes colorectaux.

6. L'utilisation selon la revendication 5, dans laquelle le médicament est préparé comme adjuvant pour des cytostatiques.

7. Paquet de médicaments comprenant une hyaluronidase selon la revendication 1 et une cytostatique ou plusieurs cytostatiques.

8. Paquet de médicaments selon la revendication 7, dans lequel la cytostatique est choisie à partir du groupe comprenant le perfosamide, le cyclophosphamide, la lomustine, la méchloréthamine, la prednisone, la vincristine, le méthotrexate, la procarbazine, le peptochémio, la doxorubicine, la vindésine, le cisplatine et la mitomycine.

9. Paquet de médicaments selon la revendication 7 ou 8, dans lequel la cytostatique est choisie à partir du groupe des antagonistes de la pyrimidine, en particulier la gemcitabine.
